# EUROPEAN PATENT SPECIFICATION

(11) **EP 2 024 011 B1**
(45) Date of publication and mention of the grant of the patent: **13.10.2010**
(21) Application number: 07761610.0
(22) Date of filing: 30.04.2007
(51) Int. Cl.: A61N 1/05

(54) **IMPLANTABLE MEDICAL LEADS WITH FLEXIBILITY AND EXTENSIBILITY, AND HAVING A SUBSTANTIALLY TWO-DIMENSIONAL NATURE**
IMPLANTIERBARE MEDIZINISCHE BIEGSAME UND DEHNBARE ELEKTRODEN MIT WEITGEHEND ZWEIDIMENSIONALER BESCHAFFENHEIT
FILS ÉLECTRIQUES MÉDICAUX IMPLANTABLES, FLEXIBLES ET EXTENSIBLES, PRÉSENTANT UNE NATURE ESSENTIELLEMENT BIDIMENSIONNELLE

(30) Priority: 28.04.2006 US 413440
(43) Date of publication of application: 18.02.2009
(73) Proprietor: Medtronic, Inc., Minneapolis, MN 55432-5604 (US)
(72) Inventor: CROSS, Thomas E., Jr., St. Francis, Minnesota 55070 (US); WILLIAMS, Michaelene M., Fridley, Minnesota 55432 (US)
(74) Representative: Hughes, Andrea Michelle
(86) International application number: PCT/US2007/067835
(87) International publication number: WO 2007/127998

(56) References cited:
- WO-A-2004/035133
- US-A- 4 154 247
- US-A1- 2005 203 599
- US-B1- 6 843 870

## Description

### Technical Field of the Invention

The present invention relates to implantable medical leads for connection between a stimulating control device and one or more stimulation or sensing electrodes, and more particularly to implantable medical leads for use in the body of a living subject that are flexible and extensible to accommodate body articulations and other movements.

### Background of the Invention

Systems and methods for electrical stimulation of electrically excitable tissue within the body of a living subject have been developed utilizing stimulating electrodes and a signal generator or control device to supply electrical charges in a controlled or predetermined manner. Such systems and methods have been developed specifically based upon a desired condition, such as to alleviate pain or to stimulate muscle movement, and based upon the application within a subject's body.

For bodily applications where the alleviation of pain is the goal, one or more stimulating and/or sensing electrodes can be implanted within nerve tissue, the brain or spinal cord for blocking pain sensation by electrical stimulation. For muscle tissue stimulation, a stimulating electrode can be implanted in the muscle tissue, whereby electrical current that is typically provided as pulses can cause muscle tissue reaction that may be controlled to cause movement of a subject's body part. Sensing electrodes are used for determining actions of the body.

Signal generators can determine when, how long and the amperage of current pulses that are to be applied for the specific application and often include hard-wired circuitry, a microprocessor with software and/or embedded logic as the controlling system for determining current pulses. In situations where temporary tissue stimulation is desired to alleviate pain or cause a temporary reaction, the electrodes can be implanted through the subject's epidermal layer and the signal generator can be utilized externally from the subject's body. Such signal generators may also be implanted within the subject's body, and typically, such an implantation is done to position the signal generator close to the stimulating and sensing electrodes with interconnecting medical leads for conducting current pulses to and from the stimulating and sensing electrodes. Implantable medical leads and externally utilized leads for these purposes are typically insulated conductors with conductive terminations at both ends for electrical connection with the signal generator and electrode. Implantable medical leads further have requirements for safe interbody use such as tissue compatibility, surgical procedure dynamics, and body fluid accommodation.

Signal generation and muscle tissue stimulation systems have more recently been developed for more complex control of a subject's bodily actions. To accomplish more complex movements, it has been developed to control a pattern of stimulation of multiple electrodes that are provided to stimulate action of distinctly different muscles in series. The attempt of such systems is to stimulate muscle tissue in the order of movement that reflects normal body movements that may have been lost or disabled by trauma or disease, the purpose of which may be to reteach a subject of a particular movement or to supplement or replace the subject's control of such movement.

A particularly complex muscular control concept has been recently developed for the purpose of reteaching a subject how to swallow, the condition of inability to swallow being known as dysphagia, which condition is a common complication with diseases such as stroke, neurodegenerative diseases, brain tumors, respiratory disorders, and the like. Dysphagia is of great concern in that the risk of aspiration pneumonia, which inflicts a 20% death rate in the first year after a stroke and 10-15% each year thereafter, is very high. Prior treatments for dysphagia required either temporary feeding through a nasogastric tube or enteric feeding through a stoma to the stomach in chronic cases.

Techniques and methods of stimulating muscles within the neck region of a human subject for the purpose of causing specifically determined muscles to react as a swallowing effect are described in PCT Publication No. WO 2004/028433, having a publication date of April 8, 2004. Specifically, by implanting electrodes in two or more muscles of the upper airway musculature and connecting the electrodes with a signal generator that provides coordinated control signals, a swallowing action can be induced in the subject's body. A goal of such technique is to reteach the subject how to swallow without such stimulation subsequent to such treatment. Other specific techniques and methods are also disclosed in U.S. Patent Nos. 5,725,564, 5,891,185, 5,987,359, 6,104,958, and 6,198,970, all to Freed et al.

One method to treat dysphagia is to electrically stimulate four primary muscles that are associated with swallowing, being the geniohyoid, mylohyoid, thyrohyoid, and hyoglossus muscles in a determined sequence as controlled by a signal generator.

In each of the techniques to cause a swallowing action described in the above prior art references, a signal generator is programmed to send electrical signals to the multiple stimulating electrodes as implanted in the appropriate muscle tissue. The pattern of electrode stimulation is set forth in the signal generator programming. Signal generators may be programmed prior to implantation, but are known to be reprogrammable through radio waves or the like. The signal generator itself is implanted within the upper pectoral chest region of a human subject as electrically connected to implanted stimulating and sensing electrodes by medical leads so that electrical signals comprising timed current pulses of predetermined amplitude and sensing signals are conducted to and from the electrodes.

The use of multiple electrodes on each side of the neck region of a human subject require the running of multiple leads along the neck and all the way to the upper region of each side of the subject's neck from the subject's chest. However, in attempting to implant and run multiple leads along the neck within neck tissue layers, the subject's head and neck must be allowed to assume movements that are associated with the swallowing action and desirably also to permit full normal head and neck movements. A human subject's head and neck includes movements having comparatively great degrees of freedom within the human body. The atlantoocipital joint, between the cranium and C1 cervical vertebrae, allows the head to tilt forward and backward (flexion and extension). The atlantoaxial joint, between C1 and C2 vertebra, facilitates rotation of the head. Lateral motion of the head is accomplished by the two sternocleidomastoid muscles and the vertebral joints.

Medical leads themselves typically comprise a conductor within an insulating cover with conductive terminations at the ends for electrical connection to components, which for treating dysphagia would be the signal generator and stimulating and/or sensing electrodes. Such leads are also typically flexible along their length, but are limited in extension by the length of the lead. As such leads are limited in extensibility, certain movements can cause one or more leads to be tensioned, the effect of which is to limit further head or neck movement in that direction. The need for multiple leads on each side of the neck greatly increases the potential that one or more leads will limit certain movements of the subject's head or neck.

While providing extra length or slack in a lead's length as it is connected between a signal generator and an electrode could potentially provide for increased movement, the flexibility of such lead would initially and uncontrollably allow lead portions to sag or collect within body cavities, spaces between tissue layers or the like. Moreover, if lead slack were to gather in a body cavity or between tissue, lead extension may then be limited or uncomfortable as the lead may slide or be pulled through tissue layers or from a body cavity during a subject's head or neck movement. Resultant discomfort and/or pain can have the effect of limiting the subject's normal movements, as a subject would tend not to do uncomfortable movements. Also, after a lead is implanted for some time, the lead begins and gradually adheres to one or more of the adjacent tissue, particularly where a sag or collection of excess lead would find itself. Then, the extra length of any such lead would not be available to permit any extension.

Also, the provision of multiple leads increases the possibility of discomfort to a subject during head, neck, or swallowing movements or otherwise. Running multiple leads along a plurality of routes to reach the necessary muscle tissue to stimulate a swallowing action adds to the possibility of subject movement limitations and/or pain or discomfort.

Document WO-A-2004/035133 represents the most relevant prior art.

The invention is an implantable medical lead as defined in claim 1.

Methods of making such implantable and extensible medical leads may comprise the steps of providing a conductive element having a length extending between first and second conductive lead terminations and including an insulating material or other lead body substantially covering the conductive element between the first and second lead terminations; and shaping the lead body and the conductive element in a non-linear manner with a shaping element by positioning and connecting the shaping element to the lead body and/or the conductive element, the shaping element being elastically deformable to permit the conductive element and lead body to be extended and to return to the shape provided by the shaping element.

A method of using such an implantable and extensible medical lead may comprise the steps of electrically connecting the medical lead between an electrode and a control device; implanting at least the medical lead and electrode within a subject's body, the electrode being further implanted within tissue to be stimulated or where sensing is desired; and stimulating an electrode from the control device by way of the medical lead.

### Brief Description of the Drawings

Fig. 1 is a plan view of a medical lead illustrated as a lead shaped over an extension thereof as a repeating sigmoid pattern;

Fig. 2 is a cross sectional view of the lead of Fig. 1 showing a shaping element provided as a tubular structure incorporated into a lead construction;

Fig. 3 is a cross sectional view of the lead of Fig. 1 showing a shaping element provided as an elongate element incorporated into another lead construction;

Fig. 4 is a cross sectional view of the lead of Fig. 1 showing a shaping element provided as a tubular structure surrounding a plurality of conductors therein and as incorporated into a another lead construction;

Fig. 5 is a cross sectional view of the lead of Fig. 1 showing a shaping element provided as an elongate element combined with a plurality of conductors and as incorporated into another lead construction;

Fig. 6 is a plan view of a medical lead in accordance with the present invention illustrated as comprising a plurality of lead bodies as part of a lead that is shaped over an extension thereof as a repeating sigmoid pattern;

Fig. 7 is a cross sectional view of the lead of Fig. 6 showing a plurality of lead bodies, each with a conductor therein, and as connected together into another lead construction;

Fig. 8 is a cross sectional view of the lead of Fig. 6 showing a plurality of lead bodies with one lead body maintaining a shaping element provided as a tubular structure with a conductor therein combined with another lead body without a shaping element, and as incorporated into another lead construction;

Fig. 9 is a cross sectional view of the lead of Fig. 6 showing a plurality of lead bodies and with a shaping element provided as an elongate element extending along a conductor within one lead body combined with another lead body without a shaping element, and as incorporated into another lead construction;

Fig. 10 is a cross sectional view of the lead of Fig. 6 showing a plurality of lead bodies adhered together with a shaping element provided as an elongate element extending along with a conductor within one lead body combined with another lead body that also maintains a similar elongate shaping element, and as incorporated into another lead construction;

Fig. 11 is a cross sectional view of the lead of Fig. 6 that is similar to the lead construction of Fig. 10, but illustrating an alternative manner of combining plural lead bodies together by thermal bonding;

Fig. 12 is a cross sectional view of another lead including showing a plurality of lead bodies and a shaping element provided as an elongate element extending along with a conductor within one lead body combined with another lead body maintaining a tubular structure as a shaping element within which a plurality of conductors are extended, and as incorporated into another lead construction;

Fig. 13 is a plan view of another medical lead illustrated as including a single lead body shaped over an extension thereof as a repeating sigmoid pattern, but with a shaping element comprising an elastic sheet material for holding the lead body in the repeating sigmoid pattern;

Fig. 14 is a partial longitudinal cross sectional view of the lead of Fig. 13 showing the shaping element provided as a sheet of elastically deformable material adhered to the lead body as it is arranged in the repeating sigmoid pattern;

Fig. 15 is a plan view of yet another medical lead in accordance with the present invention illustrated as a comprising a plurality of lead bodies that are shaped over a portion of an extension thereof as a repeating sigmoid pattern, which lead includes a plurality of branching points defining a substantially two-dimensional lead portion is a flat bundle of lead bodies, a substantially two-dimensional lead portion as a sub-bundle of lead bodies and plural individual lead portions having single lead bodies;

Fig. 16 is a cross sectional view of the lead of Fig. 15 showing a plurality of lead bodies adhered together, with some of the lead bodies maintaining a shaping element provided as a tubular structure extending along a conductor combined with a plurality of other lead bodies without a shaping element, and as arranged as a substantially two-dimensional lead portion and incorporated into another lead construction;

Fig. 17 is a cross sectional view of the lead of Fig. 15 that is similar to the lead construction of Fig. 16, but illustrating an alternative manner of combining plural lead bodies together by thermal bonding;

Fig. 18 is a schematic illustration of a plurality of branched leads leading from a signal generator as would be implanted within a human subject's chest region, the branched leads shown as would be implanted along the human subject's chest and neck to the upper neck region and terminating at points of electrical stimulation or sensing according to one possible use of the medical leads of the present invention;

Fig. 19 is a plan view of yet another medical lead in accordance with the present invention illustrated as a comprising a plurality of lead bodies as part of a lead that is shaped over a plurality of spaced portions thereof as repeating sigmoid patterns, which lead defines a substantially two-dimensional lead body bundle;

Fig. 20 is a plan view of yet another medical lead in accordance with the present invention that is customizable to create branching points and that is illustrated as a comprising a plurality of lead bodies as part of a lead that is shaped over a plurality of spaced portions thereof as repeating sigmoid patterns, which lead defines a substantially two-dimensional lead body bundle;

Fig. 21 is a cross sectional view of the lead of Fig. 20 showing lines of weakness as may be provided by a score line in connecting material provided between adjacently connected lead bodies;

Fig. 22 is a plan view of the medical lead of Fig. 20 after having been customized to create a plurality of branching points by separation of lead bodies from one another and that is illustrated as a comprising a plurality of lead bodies as a bundle, a sub-bundle, and a plurality of individual portions;

Fig. 23 is a perspective view of a medical lead assembly comprising a pair of leads branched from one another and with each lead having lead bodies for routing and branching conductors;

Fig. 24 is a side view of a lead of Fig. 23 illustrating a branched construction for multiple conductors and to permit the distal ends of conductors to be movably positioned relative to one another;

Fig. 25 is a cross-sectional view of the lead of Fig. 24 showing a pair of lead bodies combined together with the multiple conductors and a tubular shaping element extending within one lead body and an elongate shaping element extending within the other lead body;

Fig. 26 is a cross-sectional view of a separation element for selectively routing conductors from a lead body distal end; and

Fig. 27 is a cross-sectional view of a branching element for selectively routing a conductor from a lead body.

### Detailed Description of the Invention

With reference to the accompanying figures, wherein like components are labeled with like numerals throughout the several figures, medical leads and medical lead assemblies, construction methods thereof and methods of use thereof are disclosed, taught and suggested by the multiple embodiments for the purpose of providing controlled flexibility and extensibility of medical leads for implantation in a subject body. It is understood that any of the lead and lead assembly constructions described and suggested below can comprise a single lead body or multiple lead bodies, each with any number of conductors (or no conductor) and as may be provided together as leads or as a lead assembly. Moreover, medical leads and lead assemblies in accordance with the present invention have applicability for implantation in any part of a subject's body including the human body or other animals, creatures or living organisms where electrical conduction is useful. Furthermore, it is contemplated that any of the medical leads and lead assemblies are equally as useful as external or non-implanted electrical leads, although certain advantages of certain designs for implantation may be of less value for an external use application.

The present invention is described below as developed for the application of providing medical leads for implantation and use in treatments, such as for example, treatment of dysphagia, as described above in the Background section, and which treatment methods are described in greater detail in PCT Publication No. WO 2004/028433, with a publication date of April 8, 2004, as described within U.S. Patent Nos. 5,725,564, 5,891,185, 5,987,359, 6,104,958, and 6,198,970, all to Freed et al, and as described in U.S. Patent Application Serial No. 11/611,365, filed December 15, 2006, and entitled "Method and Apparatus for Assisting Deglutition."

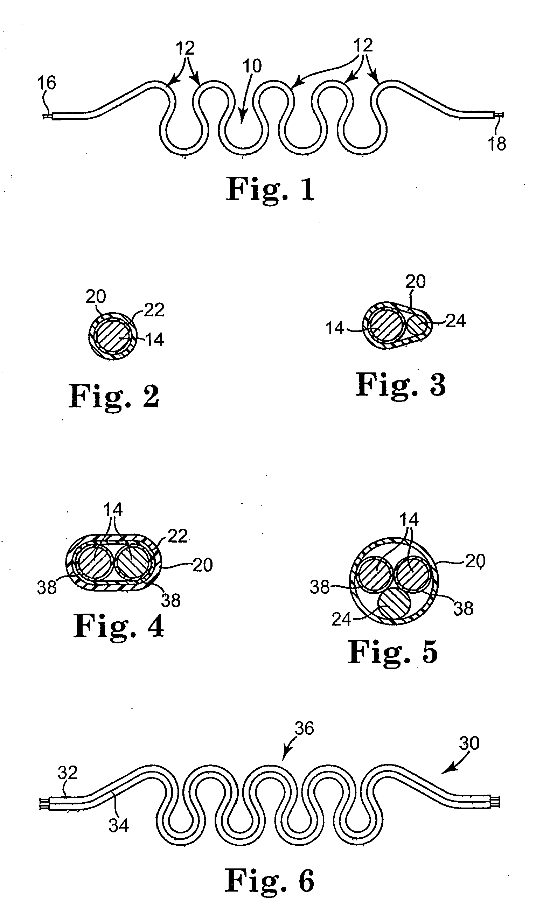

With reference initially to Fig. 1, a medical lead 10 is illustrated having a length of extension, at least a portion of which comprises a shaped portion 12. The term "shaped" means that the portion under a no-load condition will assume the desired or pre-determined shape, but which shape is elastically deformable under load and will return to the no-load shape once the load is removed. The purpose of allowing the shaped portion(s) 12 to deform elastically is to preferably provide for controlled extensibility to be designed into the medical lead 10 under any expected load for conditions that may be present under any specific application. In a general sense, it is preferable to maximize the extensibility of a lead while minimizing the load force required to cause extension. By providing a series or pattern of shaped portions at specific locations along the extension of the medical lead 10 or substantially all of the length of extension of the medical lead 10, controlled extensibility of the medical lead 10 can be locally permitted where needed under a local strain or load situation. Moreover, where the shape of the shaped portion(s) 12 may, over time, adhere with an adjacent tissue layer or layers, an aspect of the present design is that the shaped portion(s) 12 can and will move with the tissue, such as a muscle layer, without having to slide along the tissue. The medical lead 10 is preferably designed to minimize sliding and permit controlled movement with tissue, although sliding may occur. Moreover, any one or any number of shaped portions 12 can deform based upon demand under a local strain or load that may be applied to the medical lead 10 in situ after implantation. For example, with reference to the Background section, head and neck movements have been found to cause local strain and load on medical leads after implantation from normally expected head tilt and rotational movements of a subject and from a swallowing action, and muscles of the head and neck may be stimulated.

In order to obtain a desired shaping, it is important not only to create and hold the desired shape, but also to minimize stiffness to the medical lead shaped portion 12. In other words, it is also preferable to allow the lead to extend under low load. Such characteristics are preferable for implantation along a neck region of a subject, such as for treatment of dysphagia, where a target point for extensibility is around 40% when subjected to a load force of 0.1 lbs (0.045 kg) or less, preferably less. Other applications can have very different requirements with higher or lower extensibility levels under higher or lower load values. Materials that are used in constructing the medical lead 10 and the construction itself, as discussed in greater detail below, are factors in the ability to set the desired shape and also to do so while preferably minimizing stiffness.

The medical lead 10 comprises a conductor or conductive element 14, as illustrated in Figs. 2 and 3, running the length of extension of the medical lead 10 from a first conductive lead termination 16 to a second lead termination 18. A lead body 20 defining a lumen covers the conductor 14 substantially from end to end for containing and preferably electrically insulating the conductor 14. It is understood that the material of the lead body 20 can itself comprise any number of layers, which layers may be located directly on the conductor 14 or spaced from conductor 14 and may include any number of functional layers. Preferably, as described below, the lead body 20 material is selected based upon application compatible materials and requirements for such materials. Further, the lead body 20 may or may not form or define a lumen (e.g., the lead body 20 can form a lumen within which the conductor 14 is inserted or disposed; can be formed over or about (e.g., extruded) the conductor 14; can encompass the conductor 14 within a material thickness thereof (e.g., the lead body 20 is molded to the conductor 14); etc.). The conductor 14 can comprise any known or developed conductive wire or the like that may be a solid element (e.g., shaft, coil, etc.) and/or be comprised as a stranded conductor, as such are well known. Stranded wire as usable for a conductor 14 would typically be more flexible as compared with solid wire. However, the solid wire is typically more capable of being deformed to hold a shape and can have other characteristics, such as spring-back capability, that can be useful in designing leads. The lead terminations 16 and 18 can comprise any known or developed electrical connection that may be appropriate for connection between other electronic components depending on the specific application. Lead terminations 16 and 18 may be merely uninsulated wire portions for connection with other electrical connectors, or may comprise the connectors themselves as fixed to the ends of the conductor 14 within or as part of the medical lead 10. Any number of conductors 14 can be extended through the lead body 20, as insulated from one another in a conventional manner (e.g., by insulation material coating).

As shown in Fig. 1, the medical lead 10 can comprise any number of shaped portions 12 for creating extensibility of the medical lead 10, discussed above. Any effective shape for providing extensibility is contemplated, which shapes may be formed or created along the length of the medical lead 10 at one or more locations that may be regular or not, or that may extend substantially the entire length of medical lead 10. Moreover, different shapes are contemplated along the medical lead 10 as may be applied in pattern portions at spaced locations or entirely along the length of the medical lead 10.

One aspect is the ability to create a desired shape or pattern to allow extensibility along at least a portion of the medical lead 10, which extensibility and return to shape is provided by an elastic changing of the shape or pattern of shapes as created. As above, the desired shape and manner of forming such shape is preferably chosen so as to set the desired shape to be present under a no-load condition, but to elastically deform under a given load condition. As such, setting or defining the desired shape or pattern along at least a portion of the length of the medical lead 10 should take into account the ability to form or set the construction materials of the medical lead 10 for this purpose. A combination of construction techniques and material properties can be integrated to create a balanced design providing performance aspects of low load extensibility and desired shaping.

The conductor 14 may be flexible so as not to be capable of itself defining the desired shape or pattern. Alternatively, shapability of material(s) employed in forming the conductor 14 can be used as a factor in defining a desired shape or pattern. Shaping can be provided at least in part by other material of the lead construction. Shaping may be provided by material of the lead body 20, but the lead body 20, particularly when provided as an outer layer of the medical lead 10, will often have other requirements that are desirable and that may be affected undesirably if used for shaping. For example, material of the lead body 20 may be chosen based upon feel for a particular use, such as softness, lubricity, and the like, which characteristics may be modified if used for shaping, such as where shaping is set by thermal treatment. As such, it is preferable to choose at least an outer layer of the lead body 20 for desired properties of that function, and to shape the shaped lead portion 12 by a functionally distinct shaping element.

A shaping element can be provided as illustrated in Fig. 2 as an internal tubular structure 22 within a lumen defined by the lead body 20, with the conductor 14 disposed within the tubular structure shaping element 22. In Fig. 3, a shaping element is illustrated, alternatively, as an elongate shaping element 24 that is positioned together with the conductor 14 within a lumen defined by the lead body 20. In the case of either the tubular shaping element 22 or the elongate shaping element 24, which may be used in combination or selectively over different length portions of the medical lead 10, the shaping element should run along the conductor 14 over sufficient length or length portions of the lead 10 to be able to effectively define the desired shaping and pattern of shapes.
The shaping element is connected (e.g., operatively coupled) with the one or more conductors 14 as they preferably functionally extend and retract together, although physical connection is not required between the lead body 20, the selected shaping element(s) 22 or 24, and any of the one or more conductors 14 within the lead body 20. Any number of other layers, elongate elements, insulators, and the like are also contemplated in combination within or outside of the material of the lead body 20. Moreover, more than one shaping element or plurality of types of shaping elements are contemplated to be integrated together with one or more conductors 14 or with multiple lead body designs, discussed below. A shaping element such as the elongate shaping element 24 can have any cross-sectional shape, and may be provided within the lead body 20 or external thereto. Likewise, a shaping element, such as a tubular structure shaping element 22, may comprise multiple layers with some or all layers internal or external to the lead body 20.

It is a preferable construction for the medical lead 10 to have material for the lead body 20 selected based on desired properties that are suitable for implanting within a subject's body, as such properties or characteristics are known. For example, silicone rubber is desirable as an external lead body layer for the implantable medical lead 10, although any material that is determined to be implantable within a subject environment is contemplated. It is also preferable that the material of the lead body 20 not be modified significantly during a shaping process, as may be conducted based upon thermal treatment of portions of the medical lead 10 to define one or more shaped portions 12. Other known or developed manners of setting a particular material to a desired shape and from which the desired shape is elastically deformable are contemplated as well.

Materials suitable for the shaping the shaped portions 12 are preferably chosen to be sufficient to at least partially define, set and maintain a desired shape, and more preferably to do so at a minimal stiffness to permit the shape to be elastically deformed easily under load.

It is preferable to use a material as a shaping element, that can be provided as one or more tubular structures (e.g., the tubular shaping element 22 of Fig. 2) or one or more elongate elements (e.g., the elongate shaping element 24 of Fig. 3), and that can be thermally set at a temperature below a temperature that would significantly modify the material of the lead body 20, such as below a softening temperature of the material of lead body 20. The selected shaping element more preferably comprises material that softens and is deformable and shapeable at such a suitably low temperature relative to corresponding properties of the material of the lead body 20 and that, when cooled, sets or maintains the deformed shape. After forming the selected shaping element(s) to a desired shape or pattern, the shaping element 22 or 24 is preferably elastically deformable in its shape under a load force so as to permit medical lead extension as desirable for any particular application. Also, it is preferable that the shaping element 22 or 24 provide at least partial control of a shaped extensibility aspect of the lead 10, which aspect may also include contributions by the conductor 14 or other construction techniques described below.

Suitable materials for the shaping element 22 or 24 include polymeric materials and metals having characteristics described above. Thermoplastic and thermoset polymeric materials are preferable where a thermal treatment is utilized in defining the shaped portions 12 to create patterns within the medical lead 10. A preferred example for the shaping element 22 or 24 comprises urethane material, which has the ability to be thermally formed without adversely affecting a silicone rubber-type lead body 20, and which is elastically deformable at minimal loads for providing extensibility of the medical lead 10.

Shaping of any shaping element 22 or 24 with thermoset capability can be conducted by simply bending a lead portion to be patterned after providing sufficient heat from any heat source or thermal transfer device (based upon the material properties) to allow a deformable softening of the shaping element 22 or 24. Patterns can be created by using mandrels, other shaped surfaces or the like, or a mold can be utilized after or during the heating process that defines the desired pattern. For example, a mold cavity with a repeating sigmoid pattern of sufficient length can be provided and the flexible lead or lead assembly can be routed through the pattern of the mold. Then, a sufficient application of heat can soften and permit any one or more provided shaping elements to form and set with a newly set memory position based upon the shape or pattern of the mold cavity. Heat can be transferred to the lead by way of the mold or otherwise. Cooling to set the pattern can also be provided while within the mold cavity or otherwise as may be permitted under ambient conditions or by heat exchange with a cooling source. Then, with the shaping element(s) set at the desired pattern, elastic deformation of the pattern shape can allow extensibility of the medical lead 10 (or lead assembly described below).

As noted above, the one or more conductors 14 within the lead body 20 can also contribute to the pattern shaping. Conductive metals are easily deformable by applying a bending or shaping force as may be facilitated by shaped surfaces or mold-type cavities. A desirable characteristic of a conductor material comprises the ability to be deformed into the desired shape but to do so with the same amount of spring-back force tending to extend the pattern shape. Malleability of the conductor material preferably permits the desired shaping with a spring-back quality, as such ability is understood within metal bending methods and techniques. As such, a balance between a spring-back force from the one or more conductors 14 that tends to cause lead extension with resistance to elastic deformation and lead extension caused by the one or more shaping elements 22 and/or 24 can be selected to optimize lead performance.

Referring to Fig. 1, the shaped portions 12 create a repeating sigmoid pattern, which pattern is preferable to provide desired extensibility to the medical lead 10. When looking at a line connecting the lead terminations 16 and 18 as the medical lead 10 is arranged overall linearly, portions of the shaped portions 12 extended similarly from both sides of the line. This design provides a balanced extensibility. Other shaped portions 12 for creating one or more patterns other than a sigmoid pattern are contemplated, with it being preferable that the pattern minimizes sharp bends that have the effect of stiffening the pattern created by the shaped portions 12. Curved shapes are preferred, and a sigmoid pattern provides such curved shapes while effectively maximizing the amount of extensibility that can be provided to the medical lead 10.

As shown in Fig. 4, a plurality of the conductors 14 are combined within the lead body 20 and further are provided together within a tubular shaping element 22. The shaping element 22 would preferably extend over a sufficient length to create the patterns 12 of at least a portion of the lead 10 in Fig. 1. Where multiple conductors 14 are run together and in close proximity to one another, conventional insulation layers 38 are provided as needed around each conductor 14. Fig. 4 represents the ability to shape a plurality of conductors 14 with a shaping element that is provided as a tubular structure 22 within the lead body 20. Any number of such conductors 14 can be provided in this manner to create a medical lead 10 with a greater number of electrical connections.

Fig. 5 shows plural conductors 14 also provided together within a lead body 20, and with each conductor 14 insulated from one another at layers 38. An elongate shaping element 24 is illustrated as positioned to run adjacent to the conductors 14 over a sufficient length to create the pattern(s) 12 of at least a portion of lead 10 in Fig. 1. Elongate shaping element 24 is illustrated positioned to the side of the combination of conductors 14, but may otherwise be positioned, such as along and in-plane with the combination of conductors 14. Again, any number of such conductors 14 can be provided in this manner to create a medical lead 10 with a greater number of electrical connections.

Referring to Fig. 6, a medical lead 30 is illustrated comprising a plurality of lead bodies 32 and 34 that are combined together to extend between lead terminations as are suitable for electrical connection between plural electrical components, such as a plurality of electrodes as used, in one non-limiting example, in stimulating and sensing muscles for treatment of dysphagia, discussed above. The lead bodies 32 and 34 are preferably arranged side-by-side substantially along the extension of the medical lead 30, and as such create a substantially two-dimensional medical lead 30. Any number of such lead bodies can be combined to create the medical lead 30, with it being preferable to do so in the manner of extending the structure as a substantially two-dimensional assembly. That is, any number of lead bodies can be combined, and are preferably combined, by continuing the side-by-side approach on either side of the lead bodies 32 or 34. A pattern portion 36 of the lead 30 is illustrated as comprising portions of each of the lead bodies 32 and 34 that are shaped in the preferable sigmoid pattern or other pattern, as discussed above. As illustrated, it is also preferable that the pattern of pattern portion 36, whether sigmoidal or not, also extend (as compared with linear extension) in a similar two-dimensional manner with respect to the two-dimensional nature of the combination of multiple lead bodies including at least the lead bodies 32 and 34. A significant advantage of creating the lead structure and the extensibility pattern in a similar two-dimensional manner is the ability for the medical lead 30 to be usable as an implantable lead assembly that is easy to position between tissue layers of a subject's body. By this design, any number of lead bodies, each with any number of conductors and/or shaping elements disposed therein can be combined as a medical lead 30 that can be inserted between adjacent tissue layers, which multiple lead bodies 32, 34 (and potentially others) are extensible, as described above, by the provision of the pattern portion 36.

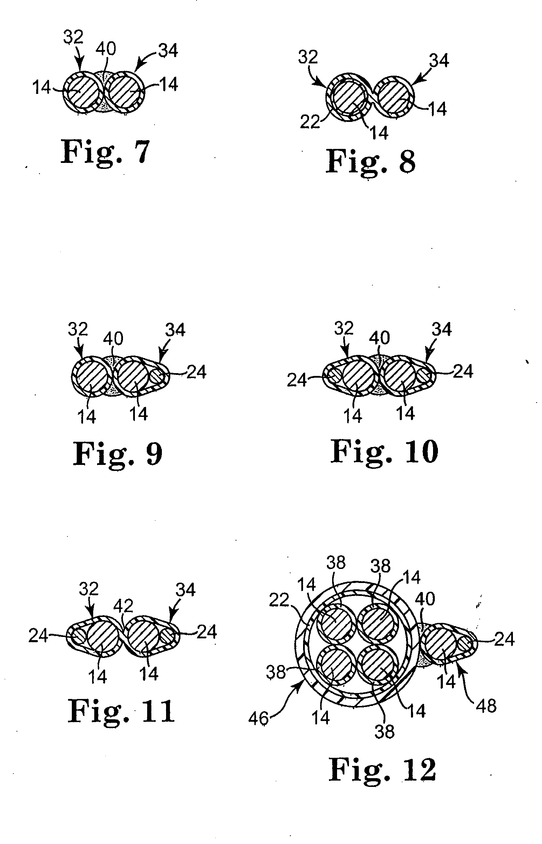

Fig. 7 illustrates a combination of multiple lead bodies 32 and 34 that are created distinctly from one another and combined by a bonding technique. One or all of the lead bodies 32 and 34 can encompass at least one conductor 14. The embodiment of Fig. 7 illustrates a combination of the lead bodies 32 and 34 using a bonding technique to shape the lead body portions without the need for a shaping element. Fig. 7 further illustrates the combination of lead bodies 32 and 34 in a side-by-side relationship over at least some of the extension of the medical lead 30 as the lead bodies 32 and 34 are bonded together. Adhesive zone 40 is shown as preferably provided adjacent to both sides of the longitudinal contact between the lead bodies 32 and 34 for combining them together. Any known or develop adhesive suitable for this purpose can be utilized, preferably having compatibility with material of the lead bodies 32 and 34 and of sufficient strength and properties for internal medical use. Alternatively, the lead bodies 32 and 34 may be thermally bonded or heat welded together along their longitudinal lengths, as such processes are also well known.

An aspect of the embodiment of Fig. 7 is the ability to use a bonding process to join adjacent lead bodies as a contributing factor to shaping the medical lead 30. As such, a step of bonding adjacent lead bodies 32 and 34 together contributes to maintaining the shapes as defined in a pattern zone 36 of a lead 30. In creating a multiple lead body lead 30, as shown in Fig. 7, the lead bodies 32 and 34, with or without conductor(s) 14, can be positioned to run side-by-side and then be shaped to a desired pattern. As above, the conductor(s) 14 can contribute to maintaining the desired shape as a result of deformation. Then, by applying the adhesive 40 along the line of contact on one or both sides, the lead 30 at its pattern 36 can be effectively set as the bonding prevents subsequent longitudinal movement of the lead bodies 32 and 34 relative to one another, which restriction maintains at least in part the desired pattern 36. Adhesive may be applied to the line of contact after shaping in a conventional manner or an adhesive may be activated to permit shaping and subsequent shaping as such techniques themselves for activating and setting adhesives such as comprising thermoplastic materials are well known. A cavity mold or the like for creating the pattern and/or facilitating heat transfer or another activating or setting parameters can be utilized as well. Bonding of the lead bodies 32 and 34 after shaping thus provides another factor that can be balanced for shaping any desired pattern with a plurality of lead bodies 32 and 34 to define a lead 30 having desired characteristics of extensibility under load.

Multiple lead bodies 32 and 34 are also illustrated in Fig. 8, each or both of which can maintaining one or more conductor(s) 14. The lead bodies 32 and 34 are shown positioned and combined in a side-by-side relationship, such as by a thermal bonding technique to connect the lead bodies 32 and 34 along a line of contact. As above, with respect to Fig. 7, thermal bonding is preferably to occur or to set, in particular, after shaping the lead bodies 32 and 34 with the desired pattern to subsequently hold them together in the shaped pattern. Fig. 8 also represents the ability to further contribute to the balancing of controlled shaping and extensibility under a desired load by incorporating a shaping element as the tubular structure shaping element 22 within at least the one lead body 32. Shaping with the tubular structure shaping element 22 and thermal bonding can be done at the same time or with the bonding after shaping in order for the bond to contribute to the shape. The Fig. 8 embodiment also represents the ability to shape medical lead 30 with multiple lead bodies 32 and 34 by the provision of a shaping element, in particular the tubular structure shaping element 22, to or within less than all of the combined lead bodies creating a medical lead 30. Fig. 9 illustrates a similar concept with the plural lead bodies 32 and 34 combined, such as by adhesive zones 40, but with only the one lead body 34 of the combination maintaining a shaping element (e.g., the elongate shaping element 24). The type of shaping element and choice to incorporate one or more shaping elements into the lead 30 design is again a matter of balancing performance characteristics of the lead 30.

Fig. 10 illustrates the possibility of combining plural lead bodies 32 and 34 with adhesive zones 40, where each lead body 32 and 34 maintains a conductor 14 and an elongate shaping element 24 within the corresponding lumen defined by the lead bodies 32 and 34. Alternatively, one or both of the lead bodies 32 and/or 34 may encompass a tubular structure 22 (Fig. 3) as shaping members usable together, or one lead body may maintain an elongate shaping element 24 with another lead body maintaining a tubular structure shaping element 22. In any case where multiple shaping element(s) are used together, at least a part of the shaping functionality results from the combination of the shaping elements being reformed or formed so as to have desired properties to provide extensibility to the medical lead 30 for a desired application. Moreover, any one or more lead bodies of a combination of multiple lead bodies may maintain shaping elements while any number of other lead bodies of the combination of multiple lead bodies may not encompass a shaping element. Fig. 11 illustrates a combination of multiple lead bodies 32 and 34 that is similar to Fig. 10 except that the lead bodies 32 and 34 are bonded as described above with respect to Fig. 8 and include a connection zone 42 of the lead body material. Such a combination of lead bodies could otherwise result from a manner of making a plurality of lead bodies in combination, such as an extrusion technique as known for encasing wiring conductors within an insulative covering, provided that the shaping elements 22 and/or 24 (any number of shaping elements 22 and/or 24) are fed along with the conductors 14 during the covering process. Such a technique, however, would not take advantage of using a bonding step as a factor in setting a pattern 36 in a lead 30. It is also contemplated to use extrusion techniques to also partially or fully form a pattern within an extruded lead body combination structure. Temperature controlled extrusion methods with distinctly controlled zones or die portions can cause same or similar materials like polymers to form differently and thus have a shaping effect that may be useful, at least in part, for making a lead construction in accordance with the present invention.

Fig. 12 illustrates a further manner of combining multiple lead bodies and one or more conductors including techniques discussed above. A first lead body 46 is combined with a second lead body 48. First lead body 46 encompasses a combination of multiple conductors 14 insulated from one another by layers 38 that are together surrounded by a tubular structure shaping element 22 and disposed within the first lead body 46. First lead body 46 is shown combined with second lead body 48 by adhesive zones 40, and second lead body 48 is illustrated as maintaining a conductor 14 and elongate shaping element 24. Fig. 12 represents the ability to combine one or more lead bodies that maintain one or multiple conductors of any number, with one or more other lead bodies maintaining one or more dissimilar conductors and/or with one or more the shaping elements. Any lead body can maintain any multiple of conductors and shaping element(s), or may encompass only conductor(s) of any number or variety or shaping element(s) of any number or variety. The construction, number of conductors and shaping elements, and materials of each component contribute to the balancing of a desired lead with extensibility properties for any particular application.

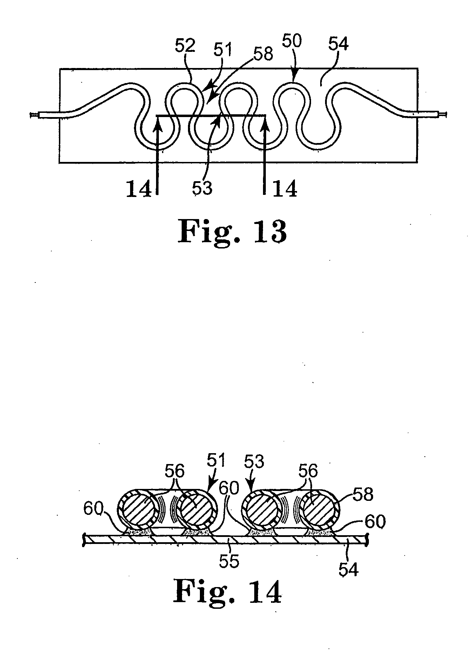

An alternative manner of shaping a medical lead is illustrated in Figs. 13 and 14. A medical lead 50 is illustrated that is similar to the medical lead 10 shown in Fig. 1, and the description of medical lead 10 and medical lead 30 with plural lead bodies and the many variations thereof as provided above are fully relevant and applicable to the embodiment of Figs. 13 and 14. However, instead of using a shaping element as a factor to contribute to shaping a desired pattern 52, an elastically extensible sheet material 54 is utilized. Shaping elements 22 and/or 24 as described above could be incorporated with the lead 50 in combination with the extensible sheet material 54. However, the extensible sheet material 54 can provide the desired shaping without the need of further shaping elements. It is further contemplated that another extensible sheet (not shown) can be similarly attached to the lead 50 on the other side from the sheet material 54 so as to create a structure with the lead 50 between the two sheets. Such a construction may be useful so that when implanted, each sheet covers the lead and can restrict fluid access around the lead.

What ever shapes or pattern are desired to be provided to the medical lead 50, the extensible sheet material 54 can define and maintain such shapes or pattern by bonding one or more lead bodies of the medical lead 50 to the sheet material 54. Bonding can be conducted by use of any adhesive that is suitable for the materials and use environment or by thermal bonding or welding the components together. Moreover, bonding is preferably performed along substantially the entire length of the medical lead 50, at least over the length of the extension of medical lead 50 within which the pattern portion 52 or plurality of such pattern portions are provided. Bonding need not be conducted continuously over any such pattern portion as may be provided by a series of bond points or zones to effectively create and maintain the desired pattern. In Fig. 14, a conductor 56 is illustrated in a partial longitudinal cross-section of the medical lead 50 as it crosses back and forth along the line of cross-section. Lead body 58 is likewise illustrated. Adhesive material 60 is further illustrated bonding the lead body 58 to the extensible sheet material 54 to maintain the pattern portion 52 with a pattern as desired, which as above may be any effective pattern permitting a desired extensibility of the medical lead 50.

In order to permit extensibility of the medical lead 50, the sheet material 54 is preferably elastically deformable to at least the degree of extensibility desired for the medical lead 50. Moreover, as with the designs discussed above, it is preferable that the medical lead 50 and thus the sheet material 54 be extensible under sufficiently low load to facilitate use as an implantable and extensible medical lead within a subject's body. So, the shaping or stiffening aspect provided by the sheet material 54 is preferably minimized to provide the desired shape under a no-load situation. Factors of the sheet material 54 for such design include properties of the material itself including its elastic deformability, the thickness of the material and the extent of which the sheet material 54 is connected to portions or all of the pattern 52 that is to desirably extend. As such, the sheet material 54 can be provided with any shape, such as illustrated that substantially operatively connects each pattern portion to one another. That is, for a pattern portion 51 to move relative to a pattern portion 53, portion 55 of the sheet material 54 would need to elastically deform as connected between pattern portions 51 and 53. If the sheet material 54 were provided as a more narrow strip or if the sheet material 54 included open areas or thinner areas, the ability to elastically deform the sheet material 54 would be changed with respect to a load force needed to obtain a desired extensibility. Otherwise, the medical lead 50 can function and be used in applications as discussed above and can be provided with any number of lead bodies and conductors to create a lead based on any of the concepts discussed and suggested above.

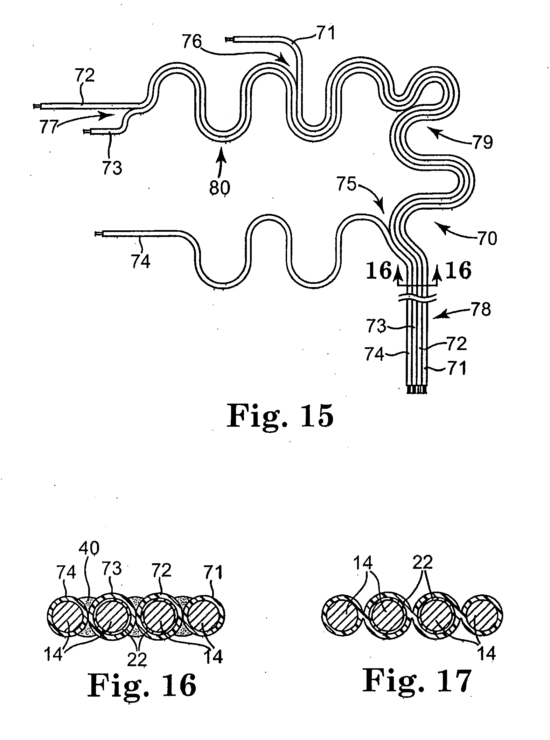

In Fig. 15, a medical lead 70 is illustrated comprising multiple lead bodies, four of which are indicated at 71, 72, 73 and 74, having a branched structure. More particularly, the medical lead 70 is shown with three branch points or junctions 75, 76 and 77 that permit one end of each lead body 71, 72, 73 and 74 (and thus any conductor(s) maintained thereby, if any) to be movable relative to the others. This construction creates a bundle portion 78, a first sub-bundle portion 79, a second sub-bundle portion 80 and ends of the individual lead bodies 71, 72, 73, and 74. In this embodiment, each lead body 71, 72, 73 and 74 maintains at least one conductor and terminations at proximal and distal ends for electrical connection between components. The advantage of such a construction is the ability to place the individual lead bodies, and thus conductor terminations, at distinctly different locations, as may be desirable for a particular application. Lead or lead assembly constructions in accordance with the principal illustrated in Fig. 15 for branching leads or conductive elements included therewith from one another as needed for a particular application can be determined to create any number of individual lead body portions or legs that are movable, sub-bundle portions of the lead bodies 71, 72, 73 and 74, and all lead body bundles. Moreover, individual lead bodies can be provided to be separate and movable from one another on either side of the length of extension of the medical lead 70.

Figs. 16 and 17 illustrate a couple of the many possible lead constructions as described and suggested above. In Fig. 16, four lead bodies 71, 72, 73 and 74 are shown bonded together as a two-dimensional bundle, with lead bodies 72 and 73 enclosing shaping elements illustrated as tubular structure shaping element 22. Fig. 17 is similar to Fig. 16, but like that shown in Fig. 11, the lead bodies 71, 72, 73 and 74 are connected, such as by a thermal bonding or welding process to provide material between adjacent lead bodies 71, 72, 73 and 74.

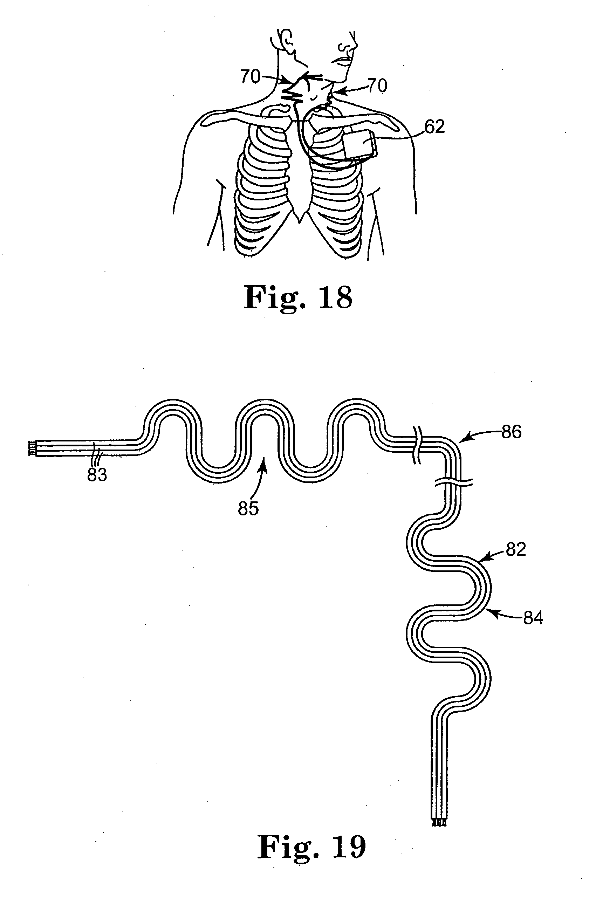

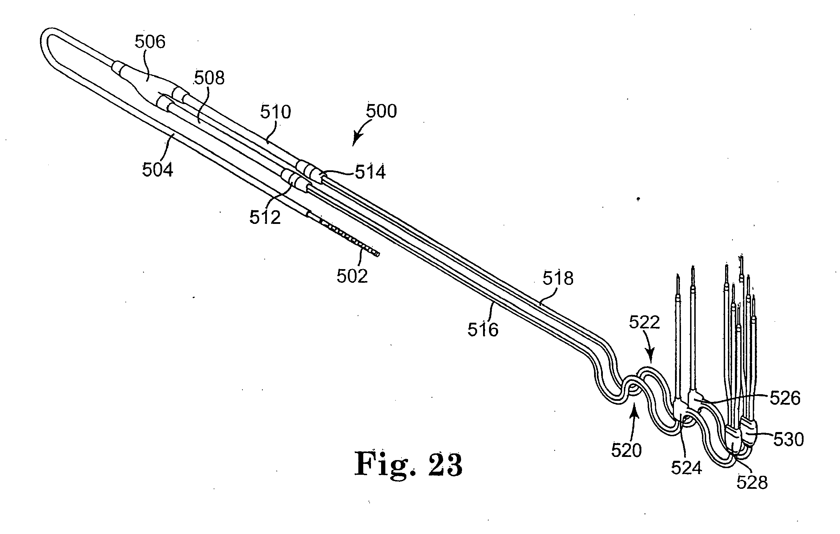

A lead assembly 500 is illustrated in Figs. 23 - 27. At proximal end 502, an electrical termination is provided, such as may be in the form of any multiple connection electrical connector or jack for electrical connection of any number of conductors to a control unit of signal generator 62 as shown in Fig. 18. Extending distally, a first tubing 504 provides a passage for any number of insulated conductors that are to be used in the lead assembly 500, which for example could be eight for treating dysphagia in accordance with one envisioned technique. A splitting element 506 separates and guides one or more conductors into second and third tubings 508 and 510. Any number of tubings can be used for a particular application and a splitter would preferably accommodate that number. For treating dysphagia in accordance with one envisioned technique, four conductors are preferably run along each guide body. At ends of the second and third tubings 508 and 510, connectors 5 12 and 514 facilitate connection to a pair of leads 516 and 518 including features. Lead 516 allows conductors to be routed along one side of a subject's neck while lead 518 allows conductors to be routed along another side of a subject's neck independently, by way of example.

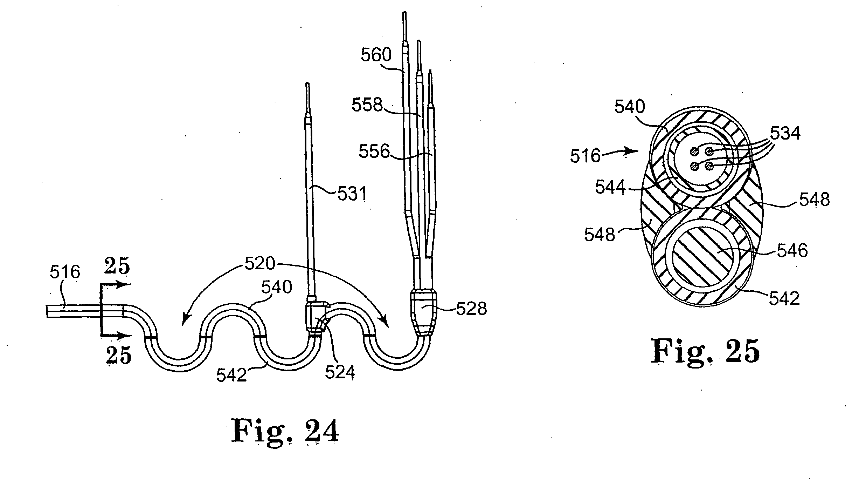

As shown in Fig. 23 with respect to both leads 516 and 518, extensibility patterns 520 and 522 provide extension of the leads 516 and 518 independently after implantation and based upon a shaped pattern, such as any shape or pattern suggested or described above. As shown in Fig. 24 with respect to lead 516, and with the understanding of similar application to lead 518, the extensibility pattern comprises a series of sigmoidal shapes as applied to a pair of lead bodies 540 and 542 that are joined together longitudinally in a side-by side relationship. As above, the pattern 520 and lead body construction share a common two-dimensionality. As shown in Fig. 25 a plurality (e.g., four) of insulated conductors 534 pass through a tubular structure shaping element 544 as a first shaping element, that in turn is disposed within the lead body 540. An elongate shaping element 546 as a second shaping element is disposed within the lead body 542. Lead bodies 540 and 542 are shown connected together by adhesive zones 548. As such, and as discussed above, the lead 516 advantageously provides the extensibility pattern and shaping as a result of the combination of a plurality of first and second shaping elements and the connection of the lead bodies 540 and 542 together to help maintain the desired shape and pattern 520. Also, by grouping the conductors 534 within the one lead body 540, deformation of the conductors 534 can be cumulatively utilized to the advantage of reducing the load to cause lead extension as a result of a spring force generated after bending the conductors 534 to the desired shape. In bending metals, it is common to bend to a degree further than desired to take out the effect of spring back. In this case, it is preferable to not do that. Then, the combination of shaping elements 544 and 546 and the connection between lead bodies 540 and 542 balances with the spring back force to define the extensibility of the lead 516 for the particular purpose.

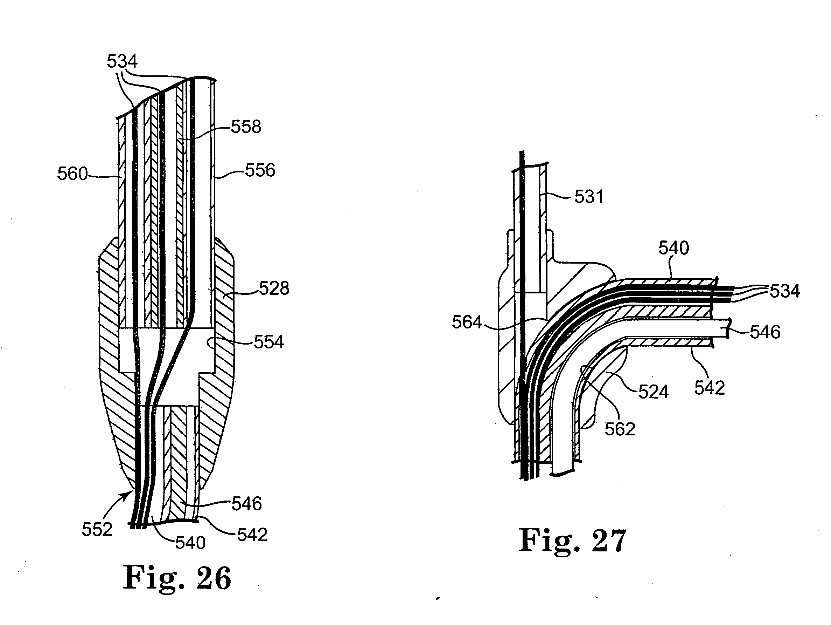

In order to provide a branched construction, an alternative manner is also illustrated in Fig. 24 than that discussed above to separate conductors and/or lead bodies from one another. A junction element 524 can be used to allow at least the lead body 540 to pass through, but also to allow a conductor from the lead body 540 to be directed into a lead body 531 for routing in accordance with the particular use. As shown in Fig. 27, the junction element 524 provides a passage 562 through which the lead body 540 is passed. A portion of the lead body material within the passage 562 is removed to permit one (or more) conductor 534 to leave lead body 540 and pass to lead body 531 that is operatively connected to the junction element 524 within a connecting passage 564. Any bonding, adhesive, or other fit technique can be used for this purpose. In this manner, both lead bodies 540 and 542 run to and beyond the junction element 524.

With reference to Figs. 24 and 26, the lead bodies 540 and 542 at distal ends thereof are operatively connected, as above, within a passage 552 of a separation element 528 that facilitates separation of one or all of the plurality of conductors 534 from lead body 540 to an internal cavity 554 of the separation element 528 that in turn permits operative connection with a plurality of further individual lead bodies 556, 558 and 560 through which at least one conductor 534 preferably passes.

Referring back to Fig. 23, this construction as applied to leads 516 and 518 provides a lead assembly that is connectible with a control device as may be implanted, as described below, and that can be flexibly run along a subject's neck (or other region) and that includes extensibility zones in each lead 516 and 518, while also providing a branched structure. As illustrated, four conductors are thus able to be effectively run along each side of subject's neck (or other region) with controlled extensibility and flexibility. The conductors 534 are connectible to electrodes as desired for stimulation and/or sensing as determined in accordance with a treatment technique under control of a control device. This construction may also minimize the number of lead bodies used for each lead 516 and 518 to two so as to minimize the volume or space required to route the leads 516 and 518 within a subject's neck. By using two leads (or more), this design takes advantage of the step of bonding plural lead bodies together as a component in balancing the extensibility shaping with a minimal load for use in the neck (or other region), along with the use of shaping element(s) (in one or more of the lead bodies) and, optionally, shaped conductors 534. Moreover, by maintaining the two (or plural) lead body construction through a branching point or junction, controllable extensibility before and after the junction is advantageously provided.

By way of but one example, in the treatment of dysphagia, discussed above, it has been found to provide such multiple conductors to multiple electrodes (not shown), as may be provided as stimulating electrodes and/or sensing electrodes, as implanted in different muscle tissue to stimulate a subject to cause a swallowing action. In particular, as illustrated in Fig. 18 schematically, it has been envisioned to implant four electrodes in different muscle tissues on each side of a subject's neck and to control stimulation of implanted electrodes by way of a signal generator 62 that can be also implanted within the subject's upper chest region to create a swallowing action. As such, a medical lead assembly, such as lead assembly 500, can be routed along the subject's neck from a signal generator 62 to four implanted electrodes (not shown) on both sides of the subjects upper neck region. The branching features incorporated within the medical lead assembly 500 provide much greater flexibility and facilitation of running the individual conductors 534 to the locations of electrodes to be implanted. For example, a branching point, such as facilitated at junction element 524, can be positioned so that individual lead body 531 (and the conductor maintained therewith) is substantially movably positionable with respect to a bundle of the lead bodies 540 and 542 after separation of one conductor 534 at junction element 524. This may allow the lead body 531 and the conductor 534 encompassed thereby to run to an electrode that is positioned substantially lower than the others within a subject's neck. The design shown in Figs. 23 through 27 and the design shown in Fig. 15 provide that the distal movable lead body portions and respective conductors can be positioned movable but relatively closer to one another, with the branching point at junction element 524 or at 75, respectively, allowing a much greater degree of freedom to the lead body 531 or 71 (and encompassed conductor(s)), respectively.

Moreover, any number of patterns or pattern portions, as described and suggested above, can be incorporated within the construction of the medical lead 70 or lead assembly 500. Shapes or patterns can be incorporated or imparted into the lead bodies individually, in connection with a sub-bundle of some lead bodies, or in convection with a bundle of all lead bodies (and the conductor(s) maintained therewith, if any). For reasons discussed above, elastic deformability of the shapes as created within the lead body bundles, sub-bundles or individual lead body portions provide flexibility and extensibility to the leads and lead assemblies, respectively. It is contemplated that a repeating pattern of similar shapes can be provided along an entire lead construction, such as the lead 70 or lead assembly 500, including as provided to any bundle portion, sub-bundle portion, and to portions of the individual lead bodies. Alternatively, different or similar patterns can be provided selectively along any portion of one or more of the leads, such as only to a bundle portion, sub-bundle portion, or individual lead body portion. A design for a particular application, such as for implanting a medical lead assembly 500 to run along a subject's neck (or other region), may dictate design criteria to the medical lead assembly 500 including not only the number of leads desired, but also the zones or portions where flexibility would be a benefit and or where other directional formations may be created and as may be controlled by subject physiology.

A branched lead 70, such as shown in Fig. 15, can be made by either combining the individual lead bodies 71, 72, 73 and 74 (or any number of two or more lead bodies) along the relevant portions thereof to create bundles, sub-bundles, or individual lead bodies at the desired locations to create branching points 75, 76 and 77 (or any number at least one), or by starting with a substantially fully combined bundle and separating the lead bodies into individual lead bodies or legs and sub-bundles as desired. Patterns can be created as described and suggested above either before or after the branched structure is created.

Preferably, for reasons also stated above, it is further desirable that the patterns created within such a branched lead 70 or a lead assembly 500 are also of a substantially two-dimensional nature discussed above and similar with respect to a preferred two-dimensional aspect of lead body combinations.

Fig. 19 illustrates a design for a medical lead 82 comprising multiple similar lead bodies 83 provided as a two-dimensional bundle without branching points. The medical lead 82 includes a first pattern zone 84 and a second pattern zone 85 that are spaced from one another along the length of extension of the medical lead 82. A corner formation 86 is illustrated to show a routing feature that may be incorporated into a medical lead or lead assembly to facilitate a particular application as may be desirable to be implanted along a determined route, that may include physiological structures or other features. For example, where a medical lead or one or more lead bodies (and maintained conductor(s)) thereof is to be routed along an articulated joint of a subject body, such a feature may be incorporated into a lead design to permit greater flexibility to the medical lead as provided by that articulated joint. It is also contemplated that instead of creating or forming such a routing feature, an extensibility pattern in accordance with the present invention can also provide such a joint flexibility in combination with extensibility, particularly where the pattern comprises a one or more curves that can also add flexibility for articulation. Moreover, features of a branched lead design as shown in Fig. 15 can be integrated with the features of the lead 82 shown in Fig. 19, any of which features can be incorporated within, or imparted into or onto, an individual lead body structure, a sub-bundle structure, or a bundle structure.

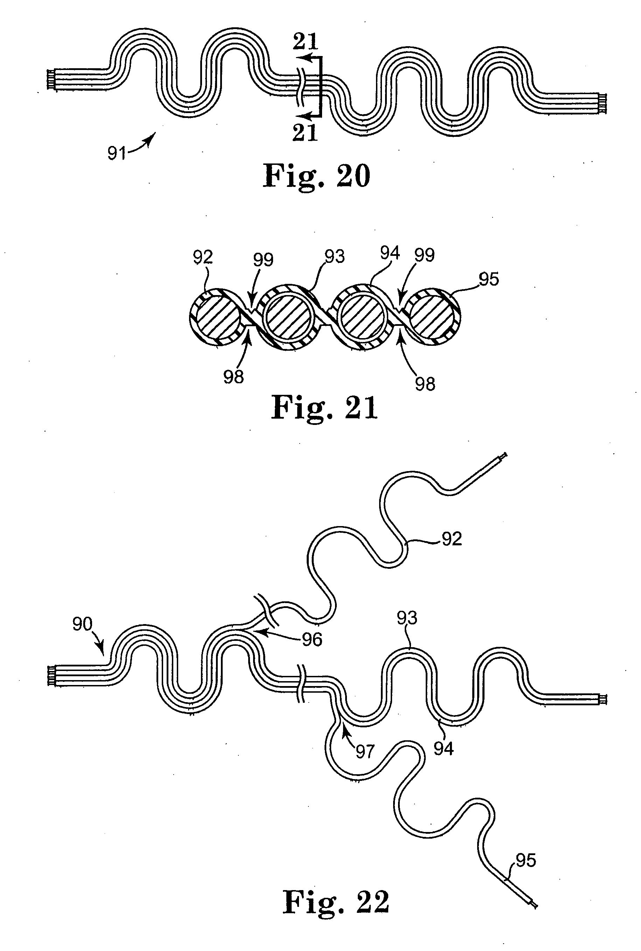

In accordance with yet another aspect of the present invention, Figs. 20, 21 and 22 illustrate a method of creating and customizing the structure of a branched lead 90 (Fig. 22) from a non-branched lead bundle 91 (Fig. 20). This concept utilizes a separation technique to create or customize the branched lead 90 starting from a non-branched lead bundle 91, particularly where the non-branched lead bundle 91 is a substantially two-dimensional combination of multiple lead bodies. Preferably also, any desired pattern portions for extensibility or other routing purposes can have been previously formed or can be created to the two-dimensional combination of multiple lead bodies in one or more similar two-dimensional oriented pattern(s).

In order to separate individual lead bodies 92, 93, 94 and 95 (and any conductor(s) encompassed therewith) as desirable to create and customize the lead 91 into the lead 90, each of the individual lead bodies 92, 93, 94 and 95 are preferably connected side-by-side to one another along individual lines of weakening that facilitate a peeling separation between any two individual lead bodies that are adjacent one another. As shown in Fig. 21, longitudinally extending connecting portions 98 can connect each individual lead body to an adjacent individual lead body. Such connecting portions 98 can comprise material as a result of thermal bonding, or may comprise added bonding material such adhesive material or material as may be used to heat weld individual lead bodies together. Along such connecting portions 98, a line of weakening can be provided to facilitate a peeling separation of individual lead bodies from one another. A line of weakening can comprise a score line as illustrated in Fig. 21 at 99, or may be created by perforations or simply by a connecting portion(s) 98 that is/are sufficiently thin to be easily broken and to permit separation of the lead bodies from one another. Alternatively, the construction of individual lead bodies themselves and a bonding of the lead bodies together to create a bundle can facilitate such peeling separation. That is, as long as the strength of any bonding technique, such as a thermal bonding or adhesive bonding, is weaker than an inherent strength of the material constructing the individual lead bodies, a separation can be facilitated. Preferably, whatever technique is utilized to provide a line of weakening, it is desirable to minimize the force required to separate or peel the lead bodies from one another.

In Fig. 22, the creation of branched lead 90 is illustrated whereby a junction or branching point 96 is created by peeling lead body 92 away from the sub-bundle of lead bodies 93 and 94, and another junction or branching point 97 is created by also peeling lead body 95 away from the sub-bundle of lead bodies 93 and 94. It is evident that a lead customization can include any number of such junctions or branching points that can be created depending on the number of individual lead bodies provided within a starting non-branched lead, such as lead 91. Moreover, the junction points can be positioned as desired for a specific application, such as discussed above with respect to Fig. 18. A further advantage of allowing such separation between lead bodies is the further customization that may be performed to adjust and create a branched lead based upon physiology or other factors of a specific subject's body, such as before or during an implantation surgery. For example, a branched lead can be created based upon measurements or other determinations of a subject's body prior to an implantation surgery and yet the lead can be adjusted before or during a surgery, such as by comparing the actual lead or lead assembly to the subject's physiology.

Uses of the leads and lead assemblies as described above and suggested in accordance with the present invention are many including internal and external connection of medical electrical components. The present invention finds particular applicability, however, for use as implanted within a subject's body and to provide what ever number of electrical connections are required, such as between a control units or signal generator 62 (Fig. 18) and any number of specifically located stimulating or sensing elements or electrodes (not shown). The present invention finds more particular applicability in the treatment of dysphagia by providing for the electrical connection of a signal generator 62 with multiple leads provided in a branched lead assembly for connection with electrodes (not shown) as located according to developed treatment methods for teaching a subject to swallow after trauma or illness reduces or eliminates such ability. Implantation surgery to facilitate implantation of medical leads and lead assemblies in accordance with the present invention include the insertion of the medical leads or lead assemblies through any one or more incisions that may be provided as part of the implantation surgery and the running of the medical leads or lead assemblies through or along tissue. As noted above, the two-dimensional nature of the preferred combination of multiple lead bodies into a bundle and the similar two-dimensional nature of one or more extensibility patterns or routing features facilitates implantation between adjacent tissue layers and permits controlled extensibility of a lead, sub-bundle or bundle as positioned between adjacent tissue layers. Furthermore, by creating leads and lead assemblies in accordance with the present invention with branching features and extensibility patterns, subject body movements can be accommodated even where the leads or lead assemblies are positioned to run near articulation points of a subject body or anywhere it is desirable for subject comfort or other reasons to permit at least one of the ends of a plurality of conductors to be relatively movable and positionable to one another.

Although the present invention has been described with reference to preferred embodiments, workers skilled in the art will recognize that changes can be made in form and detail without departing from the scope of the present invention.

## Claims

1. An implantable medical lead comprising:
a substantially two-dimensional, generally planar bundle over at least a portion of a length of the medical lead, the bundle comprising a first lead body (32) and a second lead body (34) arranged side-by-side and defining a plane of the bundle,
a plurality of conductive elements (14) disposed within at least one of the first and second lead bodies, each conductive element extending between first and second conductive lead terminations (16, 18) for electrical connection between an electrode and a control device;
wherein at least a portion of the bundle of the medical lead is extensible to increase flexibility of the medical lead by way of a non-linearly shaped length comprising a pattern that is two-dimensional substantially in the same plane as the bundle, the two-dimensional pattern including a zone of pattern portions (36) that are elastically deformable in shape to permit the extensibility of at least a portion of the medical lead, **characterized in that** the first lead body is bonded to the second lead body in said zone of pattern portions.

2. The medical lead of claim 1, wherein with respect to each of the first (32) and second (34) lead bodies, the pattern portions (36) are shaped on both sides of a line of extension connecting the respective first and second lead terminations.

3. The medical lead of claim 1, wherein the two-dimensional pattern extends along the at least a portion of the medical lead as a continuous pattern of shaped portions of the first and second lead bodies.

4. The medical lead of claim 3, wherein the continuous pattern of shaped portions comprises a repeating sigmoid pattern.

5. The medical lead of claim 1, comprising a plurality of two-dimensional patterns respective ones of which are defined at different locations along the extension of the medical lead, each of the patterns being defined at shaped portions of the first and second lead bodies.

6. The medical lead of claim 5, wherein respective ones of the plurality of two-dimensional patterns are similar to one another.

7. The medical lead of claim 6, wherein the plurality of two-dimensional patterns define a repeating sigmoid pattern.

8. The medical lead of claim 1, wherein the substantially two-dimensional define bundle further comprises a third lead body arranged side-by-side with the first lead body.

9. The medical lead of claim 1, further comprising:
a shaping element (22, 24) configured to non-linearly shape the at least a portion of the medical lead to permit extensibility of the at least a portion of the medical lead without plastically deforming the at least a portion of the medical lead.

10. The medical lead of claim 9, wherein the shaping element is shaped, when not subjected to an extension force, to define the non-linear shaped length of at least one of the first and second lead bodies, and the shaping element is elastically deformable so as to permit extensibility of at least one of the first and second lead bodies.

11. The medical lead of claim 10, wherein the shaping element comprises an elongate shaped element that extends as a tube (22) about a first one of the plurality of conductive elements, the tube and the first conductive element disposed within one of the first and second lead bodies.

12. The medical lead of claim 11, wherein the tube comprising the shaping element is provided as a separate tube from material defining the first and second lead bodies.

13. The medical lead of claim 9, wherein the shaping element comprises a thermally set material.

## Patentansprüche

1. Implantierbare medizinische Leitung, die aufweist:
ein im Wesentlichen zweidimensionales, generell planares Bündel über zumindest einen Abschnitt einer Länge der medizinischen Leitung, wobei das Bündel einen ersten Leitungskörper (32) und einen zweiten Leitungskörper (34) aufweist, die Seite an Seite angeordnet sind und eine Fläche des Bündels definieren,
eine Anzahl von leitenden Elementen (14), die innerhalb zumindest eines der ersten und zweiten Leitungskörper angeordnet sind, wobei sich jedes leitende Element zwischen ersten und zweiten Leitungssenden (16, 18) für eine elektrische Verbindung zwischen einer Elektrode und einer Steuervorrichtung erstreckt;
wobei zumindest ein Abschnitt des Bündels der medizinischen Leitung dehnbar ist, um Flexibilität der medizinischen Leitung durch eine nichtlinear geformte Länge zu erhöhen, die ein Muster aufweist, das zweidimensional im Wesentlichen in der gleichen Fläche wie das Bündel ausgebildet ist, wobei das zweidimensionale Muster eine Zone von Musterabschnitten (36) aufweist, die in der Form elastisch deformierbar sind, um die Dehnbarkeit zumindest eines Abschnitts der medizinischen Leitung zu ermöglichen, **dadurch gekennzeichnet, dass** der erste Leitungskörper mit dem zweiten Leitungskörper in der Zone von Musterabschnitten verbunden, ist.

2. Medizinische Leitung nach Anspruch 1, wobei hinsichtlich jedes des ersten (32) und zweiten (34) Leitungskörpers die Musterabschnitte (36) an beiden Seiten einer Erstreckungs- bzw. Verlängerungslinie, die die jeweiligen ersten und zweiten Leitungssenden verbindet, geformt sind.

3. Medizinische Leitung nach Anspruch 1, wobei sich das zweidimensionale Muster entlang des zumindest einen Abschnitts der medizinischen Leitung als ein kontinuierliches Muster geformter Abschnitte der ersten und zweiten Leitungskörper erstreckt.

4. Medizinische Leitung nach Anspruch 3, wobei das kontinuierliche Muster geformter Abschnitte ein wiederholendes S-förmiges bzw. sigmoides Muster aufweist.

5. Medizinische Leitung nach Anspruch 1, die eine Anzahl von zweidimensionalen Mustern, von denen jeweilige an verschiedenen Stellen entlang der Verlängerung der medizinischen Leitung definiert sind, aufweist, wobei jedes der Muster an geformten Abschnitten des ersten und zweiten Leitungskörpers definiert ist.

6. Medizinische Leitung nach Anspruch 5, wobei jeweilige der Anzahl von zweidimensionalen Mustern ähnlich einander sind.

7. Medizinische Leitung nach Anspruch 6, wobei die Anzahl der zweidimensionalen Muster ein sich wiederholendes S-förmiges Muster definiert.

8. Medizinische Leitung nach Anspruch 1, wobei das im Wesentlichen zweidimensionale definierte Bündel ferner einen dritten Leitungskörper aufweist, der Seite an Seite zu dem ersten Leitungskörper angeordnet ist.

9. Medizinische Leitung nach Anspruch 1, die ferner aufweist:
ein Formelement (22, 24), das dazu eingerichtet ist, den zumindest einen Abschnitt der medizinischen Leitung nichtlinear zu formen, um eine Dehnbarkeit des zumindest einen Abschnittes der medizinischen Leitung ohne eine plastische Deformierung des zumindest einen Abschnittes der medizinischen Leitung zu ermöglichen.

10. Medizinische Leitung nach Anspruch 9, wobei das Formelement geformt bzw. ausgeformt ist, wenn dieses nicht einer Dehnkraft ausgesetzt ist, um die nichtlineare geformte Länge zumindest eines der ersten und zweiten Leitungskörper zu definieren, und das Formelement elastisch deformierbar ist, um eine Dehnbarkeit zumindest eines des ersten und zweiten Leitungskörpers zu ermöglichen.

11. Medizinische Leitung nach Anspruch 10, wobei das Formelement ein langgestrecktes geformtes Element aufweist, das sich als Röhre (22) um ein erstes der Anzahl von leitenden Elementen erstreckt, wobei die Röhre und das erste leitende Element innerhalb eines des ersten und zweiten Leitungskörpers angeordnet sind.

12. Medizinische Leitung nach Anspruch 11, wobei das die Röhre aufweisende Formelement als eine separate Röhre bezüglich des Materials, das die ersten und zweiten Leitungskörper definiert, bereitgestellt ist.

13. Medizinische Leitung nach Anspruch 9, wobei das Formelement ein thermisches festgesetztes Material aufweist.

## Revendications

1. Fil médical implantable comportant :
un faisceau généralement plan, sensiblement bidimensionnel au-dessus d'au moins une partie d'une longueur du fil médical, le faisceau comportant un premier corps de fil (32) et un deuxième corps de fil (34) disposés cote à côte et définissant un plan du faisceau,
une pluralité d'éléments conducteurs (14) disposés à l'intérieur d'au moins un des premier et deuxième corps de fil, chaque élément conducteur s'étendant entre des première et seconde terminaisons de fil conducteur (16, 18) pour une connexion électrique entre une électrode et un dispositif de commande ;
dans lequel au moins une partie du faisceau du fil médical est extensible pour accroître la souplesse du fil médical au moyen d'une longueur formée de manière non linéaire présentant un motif qui est sensiblement bidimensionnel dans le même plan que le faisceau, le motif bidimensionnel incluant une zone de parties de motif (36) qui sont élastiquement déformables en forme pour permettre l'extensibilité d'au moins une partie du fil médical, **caractérisé en ce que** le premier corps de fil est fixé sur le deuxième corps de fil dans ladite zone de parties de motif.

2. Fil médical selon la revendication 1, dans lequel en ce qui concerne chacun des premier (32) et deuxième (34) corps de fil, les parties de motif (36) sont formées des deux côtés d'une ligne d'extension reliant les première et seconde terminaisons de fil respectives.

3. Fil médical selon la revendication 1, dans lequel le motif bidimensionnel s'étend le long d'au moins une partie du fil médical sous forme d'un motif continu de parties mises en forme des premier et deuxième corps de fil.

4. Fil médical selon la revendication 3, dans lequel le motif continu de parties mises en forme comporte un motif sigmoïde répétitif.

5. Fil médical selon la revendication 1, comportant une pluralité de motifs bidimensionnels dont des motifs respectifs sont définis à différents emplacements le long de l'extension du fil médical, chacun des motifs étant défini sur des parties mises en forme des premier et deuxième corps de fil.

6. Fil médical selon la revendication 5, dans lequel des motifs respectifs parmi la pluralité de motifs bidimensionnels sont similaires les uns aux autres.

7. Fil médical selon la revendication 6, dans lequel la pluralité de motifs bidimensionnels définit un motif sigmoïde répétitif.

8. Fil médical selon la revendication 1, dans lequel le faisceau défini sensiblement bidimensionnel comporte en outre un troisième corps de fil disposé côte à côte avec le premier corps de fil.

9. Fil médical selon la revendication 1, comportant en outre :
un élément de mise en forme (22, 24) configuré pour former de manière non linéaire la au moins une partie du fil médical pour permettre une extensibilité de la au moins une partie du fil médical sans déformer plastiquement la au moins une partie du fil médical.

10. Fil médical selon la revendication 9, dans lequel l'élément de mise en forme est mis en forme, lorsqu'il n'est pas soumis à une force d'extension, pour définir la longueur mise en forme non linéaire d'au moins un parmi les premier et deuxième corps de fil, et l'élément de mise en forme est élastiquement déformable de manière à permettre une extensibilité d'au moins un parmi les premier et deuxième corps de fil.

11. Fil médical selon la revendication 10, dans lequel l'élément de mise en forme comporte un élément mis en forme allongé qui s'étend sous la forme d'un tube (22) autour d'un premier parmi la pluralité d'éléments conducteurs, le tube et le premier élément conducteur étant disposés à l'intérieur d'un parmi les premier et deuxième corps de fil.

12. Fil médical selon la revendication 11, dans lequel le tube comportant l'élément de mise en forme est prévu sous la forme d'un tube séparé à partir d'un matériau définissant les premier et deuxième corps de fil.

13. Fil médical selon la revendication 9, dans lequel l'élément de mise en forme comporte un matériau fixé thermiquement.
